(19) Europäisches Patentamt
European Patent Office
Office européen des brevets

(11) **EP 2 494 563 B1**

## (12) EUROPEAN PATENT SPECIFICATION

(45) Date of publication and mention
of the grant of the patent:
**21.01.2015 Bulletin 2015/04**

(21) Application number: **10771617.7**

(22) Date of filing: **25.10.2010**

(51) Int Cl.:
***H01G 4/35*** *(2006.01)*        ***A61N 1/375*** *(2006.01)*
***H01B 17/30*** *(2006.01)*

(86) International application number:
**PCT/US2010/053889**

(87) International publication number:
**WO 2011/053539 (05.05.2011 Gazette 2011/18)**

(54) **CERAMIC COMPONENTS FOR BRAZED FEEDTHROUGHS USED IN IMPLANTABLE MEDICAL DEVICES**

KERAMIKTEILE FÜR GELÖTETE DURCHFÜHRUNGEN BEI IMPLANTIERBAREN MEDIZINISCHEN VORRICHTUNGEN

COMPOSANTS EN CÉRAMIQUE DESTINÉS À DES TRAVERSÉES BRASÉES UTILISÉES DANS DES DISPOSITIFS MÉDICAUX IMPLANTABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2009  US 200961256572 P**
**21.04.2010  US 764140**

(43) Date of publication of application:
**05.09.2012  Bulletin 2012/36**

(73) Proprietor: **Medtronic, Inc**
**Minneapolis, Minnesota 55432 (US)**

(72) Inventors:
• **REITERER, Markus, W.**
**Plymouth, MN 55442 (US)**
• **THOM, Andrew, J.**
**Maple Grove, MN 55369 (US)**
• **NYGREN, Lea, A.**
**Bloomington, MN 55438 (US)**
• **WOLF, Willam, D.**
**St. Louis Park, MN 55426 (US)**

(74) Representative: **Hughes, Andrea Michelle**
**Dehns**
**St Bride's House**
**10 Salisbury Square**
**London EC4Y 8JD (GB)**

(56) References cited:
**US-A- 4 678 868     US-B1- 6 414 835**

**Description**

FIELD

The present teachings relate to feedthrough assemblies that incorporate a ceramic insulator having a specified surface quality that minimizes hermetic sealing failures in implantable electronic medical devices. One example of such feedthrough assemblies is found in US 6 414 835.

SUMMARY

This section provides a general summary of the disclosure.

The present invention provide a feedthrough assembly according to claim 1, as well as a method of forming a feedthrough assembly according to claim 9, including a metallic ferrule, and a biocompatible, non-conductive, high-temperature, co-fired insulator engaged with the metallic ferrule at an interface between the ferrule and the insulator. The insulator includes a first surface at the interface and a second surface internal to the insulator. At least one conductive member may be disposed at the second surface, wherein at least the first surface of the insulator is devoid of surface cracks greater than 30 $\mu$m. The first surface of the insulator may also be devoid of a surface roughness, $R_a$, greater than 0.5 $\mu$m.

Further areas of applicability will become apparent from the description provided herein. The description and specific examples in this summary are intended for purposes of illustration only and are not intended to limit the scope of the invention as set forth with the appended claims.

DRAWINGS

The drawings described herein are for illustrative purposes only of selected embodiments and not all possible implementations, and are not intended to limit the scope of the present disclosure.

FIG. 1 depicts a schematic diagram showing in cross-section view a feedthrough assembly according to various embodiments of the present teachings;

FIG. 2 depicts a roughness chart for determining surface roughness ($R_a$) of a section of a ceramic insulator according to an exemplary embodiment of the present teachings;

FIG. 3 depicts a scanning electron micrograph of an aluminum oxide ceramic insulator showing the various surface cracks that are above the critical flaw size of 30 $\mu$m to 70 $\mu$m; and

FIG. 4 depicts a scanning electron micrograph depicting an aluminum oxide insulator having no surface cracks above the critical flaw size of 30 $\mu$m.

Corresponding reference numerals indicate corresponding parts throughout the several views of the drawings.

DETAILED DESCRIPTION

The invention refers to a feedthrough assembly of claim 1 and a method for making a feedthrough assembly of claim 9.

Example embodiments will now be described more fully with reference to the accompanying drawings.

Exemplary embodiments are provided so that the present teachings will be thorough, and will fully convey the scope to those who are skilled in the art. Numerous specific details are set forth, such as examples of specific components, devices and methods, to provide a thorough understanding of embodiments of the present teachings. It will be apparent to those skilled in the art that specific details need not be employed, that exemplary embodiments may be embodied in many different forms. In some example embodiments, well-known processes, well-known device structures and well-known technologies are not described in detail.

Applications exist where it may be necessary to penetrate a sealed container with one or more electrical leads or electrical contacts so as to provide electrical access to and from electrical components enclosed therein. One such application may be for an electrochemical cell or for an implantable electronic medical device. Such an implantable electronic medical device may comprise for example, an implantable drug pump, an implantable sensor capsule, a cochlear implant, an implantable pulse generator (IPG) such as those adapted for providing deep brain stimulation, nerve stimulation, electrical pacing therapy and cardiac rhythm management techniques (e.g., for delivering electrical stimulation therapy for various cardiac arrhythmias). In addition, such implantable electronic devices can be used to sense optical signals or deliver optical impulses for stimulation. All such devices, including discrete electrochemical cells, are intended to be covered under the rubric of implantable electronic medical devices. A typical implantable electronic medical device can have one or more housing or encasement members for isolating the active contents of an electrochemical cell (e.g., battery or capacitor) which can be coupled to one or more electrical components within and/or coupled to the implantable electronic medical device. The implantable electronic medical device typically has at least two major outer housing members that form a hermetically- sealed housing when welded together to provide a hermetically-sealed interior space for the components of the implantable electronic medical device.

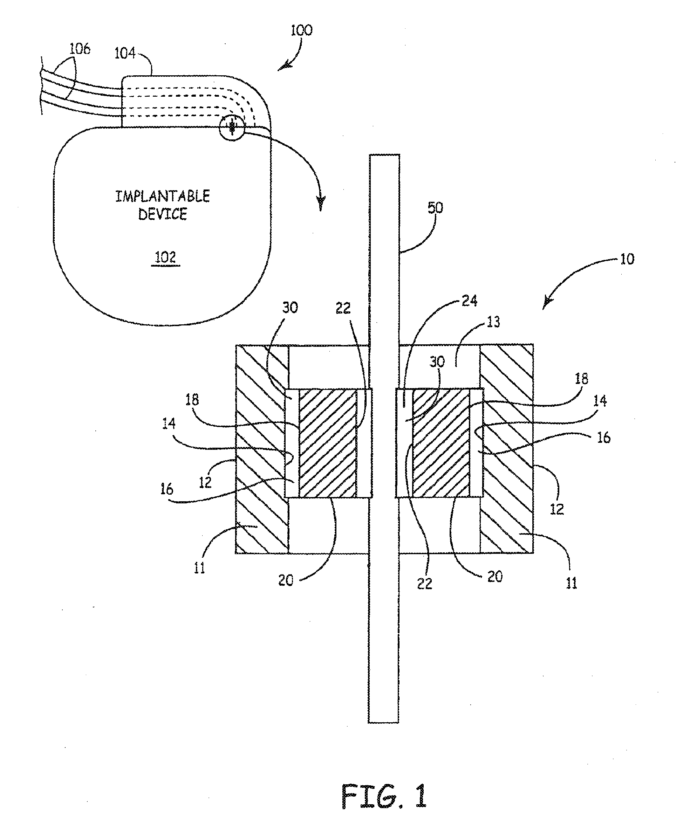

Electrical feedthroughs provide an electrical circuit path extending from the interior of a hermetically-sealed metal case to an external point outside the case while maintaining the hermetic seal of the case. FIG. 1 illustrates an exemplary electronic implantable medical device 100 incorporating a feedthrough assembly 10 according to the present teachings. Medical device 100 generally includes a medical device housing 102 having a connector module 104 coupled thereto. Connector module 104 electrically couples various internal electrical components (not shown) located within medical device housing 102 to external operational and/or diagnostic systems (not shown) located distal to device 100 through use of leads 106. Electrical connection of leads 106 to the internal electrical components is accomplished through use of feedthrough assembly 10.

An exemplary feedthrough assembly 10 according to the present teachings may include a cylindrical ferrule 11, a conductive element 50 (e.g. a pin), and a cylindrical insulator 20. Ferrule 11 includes a ferrule outer surface 12,and a ferrule lumen surface 14 that defines an aperture 13. Ferrule 11 may be brazed to insulator 20 and, therefore, is separated from insulator 20 by a ferrule-insulator braze gap 16. Insulator 20 includes an insulator outer surface 18 and an insulator lumen surface 22. Insulator 20 may be brazed to conductive element 50 and, therefore, may be separated from conductive element 50 by an insulator-conductive element braze gap 24. Braze gaps 16 and 24 are filled with braze material 30. While the exemplary embodiment in FIG. 1 shows a cross-section of a cylindrical insulator 20, a cylindrical ferrule 11, and a cylindrical conductive element 50, other shapes can be envisioned and the present teachings should not be limited thereto.

Although only a single conductive element 50 is illustrated, it should be understood that feedthrough assembly 10 may include a ferrule 11 disposed about a plurality of conductive elements 50. Moreover, other exemplary embodiments of feedthroughs are described in U.S. Patent No. 4,678,868 issued to Kraska et al. and entitled "Hermetic Electrical Feedthrough Assembly," in which an alumina insulator provides hermetic sealing and electrical isolation of a niobium electrical contact from a metal case. Further, for example, a filtered feedthrough assembly for implantable medical devices is also shown in U.S. Patent No. 5,735,884 issued to Thompson et al. and entitled "Filtered Feedthrough Assembly For Implantable Medical Device," in which protection from electrical interference is provided using capacitors and zener diodes incorporated into a feedthrough assembly. Other implantable feedthrough assemblies useful in the present teachings include those described in U.S. Patent Nos. 7,164,572, 7,064,270, 6,855,456, 6,4148835 and 5,175,067 and U.S. Patent Application Publication No. 2006/0247714.

Ferrule 11 may be formed of a conductive material and is generally adapted to secure feedthrough assembly 10 to housing 102. In some embodiments, the conductive material may be a metallic material including titanium, niobium, platinum, molybdenum, tantalum, zirconium, vanadium, tungsten, iridium, rhodium, rhenium, osmium, ruthenium, palladium, and any combination thereof. Ferrule 11 may have any number of geometries and cross-sections so long as ferrule 11 is an annular structure such as a ring with a lumen therein to hermetically seal insulator 20. In some embodiments, ferrule 11 may surround insulator 20 and provide ferrule lumen surface 14 to contact braze material 30 disposed in the ferrule-insulator braze gap 16 to form a hermetic seal.

Insulator 20 may be formed from a material including an inorganic ceramic material (e.g., sapphire), a glass and/or a ceramic-containing material (e.g., diamond, ruby, crystalline aluminum oxide, and zinc oxide), and an electrically insulative material. Insulator 20 may also be formed of liquid-phase sintered ceramics, co-fired ceramics, a high-temperature glass, or combinations thereof. Insulator 20 may also include a sputtered thin niobium coating at least at surfaces 18 and 22. Because the sputtered niobium coating is thin, the coating is not shown for illustration purposes. Insulator 20 is not limited to any particular configuration for use in feedthrough 10, so long as insulator 20 accommodates one or more electrically conductive elements 50.

Braze material 30 may be formed of a material such as gold. Other materials sufficient to braze ferrule 11 to insulator 20, and sufficient to braze insulator 20 to conductive element 50, however, are contemplated. For example, braze material 30 may include materials such as high purity gold, and gold alloys containing silver, copper, tin, and/or zinc without departing from the scope of the present teachings. Alternatively, braze material 30 may comprise a material having a melting point less than melting points of ferrule 11, conductive element 50, and insulator 20.

Conductive element 50 may be formed of materials such as iridium, molybdenum, niobium, palladium, platinum, tantalum, titanium, tungsten, and combinations thereof.

Feedthrough assembly 10 provides an electrical circuit pathway extending from the interior of hermetically-sealed device housing 102 to an external point outside housing 102 while maintaining the hermetic seal of the housing 102. The fluid tight hermetic seal is formed by metal braze 30 disposed in ferrule-insulator braze gap 16 and insulator-conductive element braze gap 24 formed between the insulator 20 and the ferrule 11 and between the insulator 20 and conductive element 50, respectively. A conductive path is provided through feedthrough 10 by conductive element 50, which is electrically insulated from housing 102.

For brittle materials such as ceramics, it is generally assumed that the most severe flaw in a stressed volume leads to failure (Weibull assumption). Because of the stress state, the origin of most fractures in thermally-stressed ceramic materials can be linked to surface flaws. Based on linear-elastic fracture mechanics, the stress intensity factor, $K$, has to be larger than the fracture toughness, $K_c$, of the material for a crack to be propagated. The stress intensity factor, $K$, depends on the flaw size and geometry, and the applied stress. Under constant stress, a crack will propagate if the size of the defect is over critical. Thus, surface quality may be seen as an essential factor for the reliability of a brittle structure, in particular, ceramic co-fired parts.

Surface quality of insulator 20 in feedthrough assembly 10 in electronic implantable medical device 100 is generally specified as surface roughness only, as it is commonly linked to flaw size. However, without wishing to be bound by theory, it is believed from a fracture and mechanical point of view, that it is not the surface roughness, but the flaw size that determines the reliability of a brazed ceramic component.

The surface quality of insulator 20, and in particular the surface quality of surfaces 18 and 22 of insulator 20 to be brazed with a liquid metal braze material 30, can be manipulated to have a surface quality that, when above a certain threshold (a critical flaw size), positively impacts hermetic sealing between insulator 20 and ferrule 11 and conductive member 50. Flaws in ceramics are commonly introduced during green processing or sintering. A second source of surface defects can be traced back to hard machining after sintering, when the component obtains its final geometry and surface finish. Hard machining is critical to the strength of a ceramic, as surface flaws can be eliminated or introduced. Experimental observations have shown that there is a general correlation between surface roughness and surface flaws. Those experiments showed that specimens with a smaller surface roughness value have higher fracture strength.

Surface flaws weaken ceramic insulator 20 and enable propagation of cracks. For feedthrough assembly applications, a hermetic seal between conductive member 50, insulator 20, and ferrule 11 is a fundamental and essential component in electronic medical device 100. Since the reliability of implantable electronic device 100 depends in large part on hermetic sealing of the components of the feedthrough assembly 10, the integrity of such seals is of paramount importance. In general, ceramic insulator 20, braze material 30, and ferrule 11 have different thermal and mechanical properties. Hence, residual stresses build up during cool-down after brazing. The sign and the magnitude of the residual stresses in the system depend on the coefficient of thermal expansion, the Young's modulus, and other thermo-mechanical properties of all involved materials.

In the exemplary embodiments of the present teachings, biocompatible, non-conductive, high-temperature insulators 20 are provided for use in feedthrough assemblies 10 used, for example, in implantable electronic medical devices 100. As stated above, medical devices 100 may include implantable pulse generators for cardiac pacemakers that provide electrical stimulation to an arrhythmic heart or neural tissue, implantable defibrillators, implantable cardioverters, implantable cardiac pacemaker-cardioverter-defibrillators (PCD), implantable chemical/biochemical sensors (e.g., glucose sensors), cochlear implants, implantable drug-medicament or metabolite delivery devices (e.g., insulin pumps), and implantable medical devices that perform *in vivo* diagnostic monitoring and telemetry. Insulator 20 can be made from an electrically non-conductive high-temperature material, preferably a ceramic material, or combination of non-ceramic materials coated with, or having an outer layer comprising ceramic materials. In exemplary embodiments, insulator 20 may comprise alumina, silica, boron nitride, diamond, glass, ruby, sapphire, zircon, zirconia, zirconia toughened alumina, silicon nitride, silicon carbide, silicon oxide, and combinations thereof. A material of special interest is co-fired alumina, where a ceramic package comprised of electrically insulating alumina and electrically conductive refractory metal such as iridium, niobium, palladium, tantalum, titanium, platinum, tungsten, molybdenum, or combinations thereof, is sintered in one common step forming a hermetic system. In such an insulator 20, the conductive element 50 need not be brazed to insulator 20 because the electrically conductive refractory metal serves as conductive element 50.

Insulators 20 of the present teachings are manufactured to have a specific surface quality. The specific surface quality is one in which the brazing surfaces 18 and 22 (i.e., brazing regions) at the insulator-ferrule interface 16 and the insulator-conductive member interface 24, has a number of cracks with specific dimensions, including any one of width, length and depth that is below a certain threshold. Having a predetermined insulator surface quality provides insulators 20 that can be used to hermetically seal the above-mentioned feedthrough assembly 10 for the electronic implantable device 100.

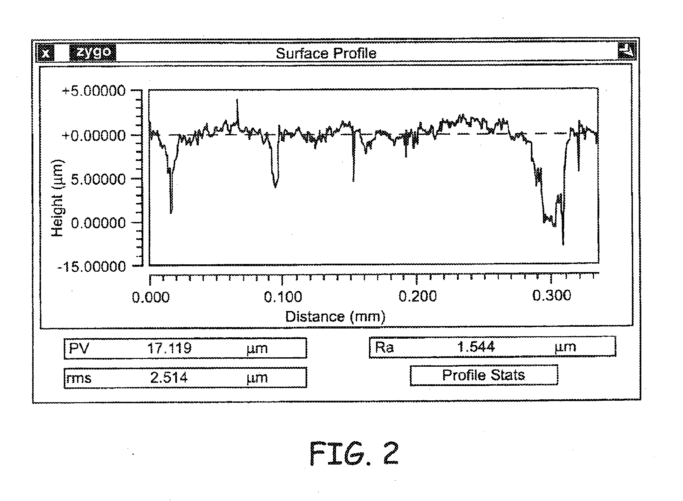

The residual stresses in a brazed joint depend on the coefficient of thermal expansion, on the Young's modulus, and other mechanical properties such as creep or relaxation behavior of the involved materials. In addition, the stress is limited by the yield strength of the metallurgical phases. Arithmetic surface roughness ($R_a$) can be determined by sampling a section of standard length from a mean line on a roughness chart. Referring to FIG. 2, the mean line is laid on a Cartesian coordinate system wherein the mean line runs in the direction of the x-axis and magnification is the y-axis. The overall scanned area was 350 $\mu$m x 250 $\mu$m, with an $R_a$ value of ~0.90 $\mu$m. An above line profile from this area gives peak-to-line values of ~17 $\mu$m, or about 20xRa. The arithmetic surface roughness may be expressed in micrometers or nanometers ($\mu$m & nm). In calculating fracture mechanics, Griffith (Griffith, A. A., "The Phenomena of Rupture and Flow in Solids. "Philosophical Transactions, Series A, Vol. 221, Royal Society of London, 1920, pp. 163-198) argued that fractures do not start from a pristine surface, but from pre-existing flaws, known as 'Griffith flaws', on that surface.

Brittle solids such as high-temperature glasses and liquid-phase or solid state sintered and co-fired ceramics are severely weakened by sharp notches or flaws in the surface because these imperfections (that are rarely visible to the naked eye) produce very high stress concentrations. The cracks and surface flaws in glasses and ceramics propagate if the stress intensity factor exceeds the fracture toughness of the material. On the basis of previous work, Griffith modeled a static crack as a reversible thermodynamic system. In the configuration that minimizes the total free energy of the system, the crack is in a state of equilibrium and, therefore, on the verge of extension. The total energy $U$ in the system is:

$$U = U_s + U_m \qquad \text{(Equation 1)}$$

where $U_m$ is the mechanical energy (the sum of the strain potential energy stored in the elastic medium and the potential energy of the outer applied loading system) and $U_s$ is the free energy expended in creating new crack surfaces. Therefore $U_m$ favors crack extension, whereas $U_s$ opposes it. The equilibrium requirement $dU/da = 0$ is known as the Griffith energy-balance concept, where $a$ is the crack length. From this, Griffith calculated the critical conditions at which instantaneous failure occurs as:

$$\sigma_f = \sqrt{2\mathrm{E}\gamma/(\pi a_c)} \qquad \text{(Equation 2)}$$

where $\sigma_f$ is the failure stress, E is the Young's modulus, $\gamma$ is the specific free surface energy, and $a_c$ is the critical crack length for crack growth.

Employing the Griffith criterion:

$$a_c = \frac{1}{\pi}\left(\frac{K_{IC}}{\sigma_f}\right)^2 \qquad \text{(Equation 3)}$$

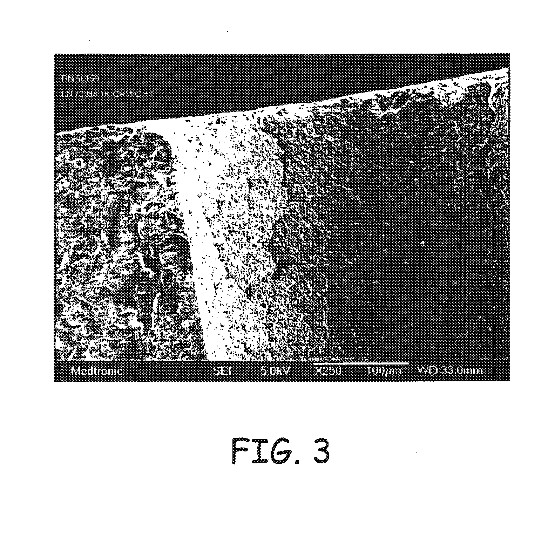

wherein $K_{IC}$ is the term for plane stress fracture toughness in megapascal times square root meter (MPa m$^{0.5}$), $\sigma_f$ defines the applied uniform stress in megapascal (MPa) and $a_c$ defines the final failure initiating crack size in meter (or the critical flaw size). Frequently, the value $\pi$ is multiplied by a geometry factor, which is close to unity. For all estimates presented below, the geometry factor is assumed to be equal to unity. For an exemplary ceramic insulator 20 comprising alumina, if a fracture toughness $K_{IC}$ = 3 MPa m$^{0.5}$ and an acting stress $\sigma$ = 300 MPa exists, a threshold consisting of a critical flaw size of $\approx$ 32 $\mu$m is calculated. This would be the critical flaw for instantaneous failure at a maximum principal stress of 300 MPa for a sharp crack. In fact, a crack smaller than the critical flaw size can propagate without causing failure until it has reached the critical size. FIG. 3 is a scanning electron micrograph of an aluminum oxide ceramic insulator showing the various surface cracks that are above the critical flaw size of 30 $\mu$m to 70 $\mu$m.

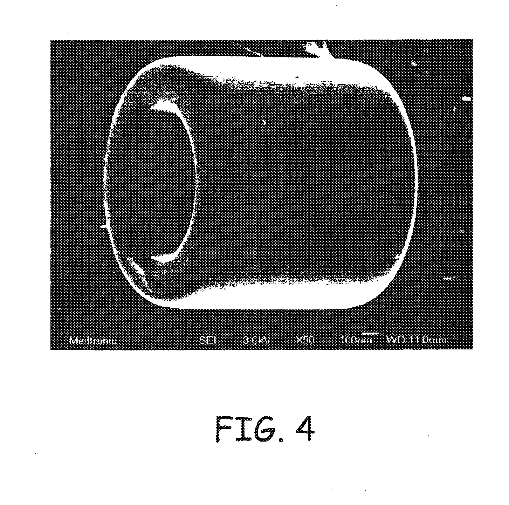

In a machined surface with a roughness $R_a$ between 0.5 and 1.5 $\mu$m, it is common to find peak to valley distances, which are one order of magnitude larger than $R_a$. It is also common to find peak to valley distances which are 2 orders of magnitude larger than $R_a$. These geometric features can act as flaws in a fracture mechanical sense. With estimated design stresses between roughly 200 and 300 MPa, and correlated critical flaw sizes between 30 and 70 $\mu$m, peak to valley distances greater than 30 or 70 $\mu$m, respectively, should be avoided. The insulators 20 of the present teachings are manipulated to have a threshold surface finish requirement for a robust braze joint; i.e., a brazing region on one or more sides of the insulator having a surface roughness $R_a$ (for any surfaces 18 and 22 of insulator 20 which are brazed) less than 1.5 $\mu$m, less than 1.4 $\mu$m, less than 1.3 $\mu$m, less than 1.2 $\mu$m, less than 1.1 $\mu$m, less than 1.0 $\mu$m, less than 0.9 $\mu$m, less than 0.8 $\mu$m, less than 0.7 $\mu$m, less than 0.6 $\mu$m, or less than 0.5 $\mu$m. FIG. 4 is a scanning electron micrograph depicting an aluminum oxide insulator having no surface cracks above the critical flaw size of 30 $\mu$m.

When comparing the insulator of FIG. 2 to the insulator of FIG. 4, it can be seen that the insulator of FIG. 4 has a considerably smoother surface relative to the insulator shown in FIG. 3. Because the insulator of FIG. 3 has cracks having lengths greater than the critical flaw size of 30 $\mu$m, the insulator of FIG. 3 has a substantially greater likelihood of a hermetic failure during the brazing process, or during use of the feedthrough assembly 10 in medical device 100. In contrast, the insulator of FIG. 4 including a smooth surface devoid of cracks having the critical flaw size of 30 $\mu$m will

satisfactorily bond to braze material 30 during the brazing process and has a substantially lesser likelihood of failure during use of feedthrough assembly 10 in medical device 100.

Methods for determining the surface characteristics of insulator 20 are well known in the art. Exemplary techniques can include confocal microscopy, electrical capacitance, electron microscopy and interferometer analysis, such as white light interferometer analysis using a Veeco WYKO NT3300 interference microscope commercially available from Veeco Instruments, Tucson, AZ, USA. Several interference measurement techniques can be used to determine the surface quality, the $R_a$ value of the ceramic insulator, and the dimensions of surface cracks, including phase shifting interferometry, vertical scanning interferometry and enhanced visual interferometry as described in Harasaki, A., et al, (2000) "Improved Vertical Scanning Interferometry," Appl. Opt. 39:2107-2115.

Biocompatible, Electrically Non-Conductive, High-Temperature Insulators

The present teachings provide feedthrough assemblies comprising high-temperature biocompatible electrically non-conductive insulator materials including high-temperature glass and liquid-phase or solid state sintered and co-fired ceramic insulators having no surface cracks located at least at the insulator brazing region operatively disposed at the interface 16 between insulator 20 and ferrule 11, and at the interface 24 between insulator 20 and conductive member 24 greater than 30 $\mu$m.

Insulators 20 comprising the surface characteristics described above can be manufactured from biocompatible high-temperature materials, including ceramics and high-temperature glasses. Some of the materials useful in the manufacturing of the ceramic insulators can include alumina, co-fired alumina, zircon, zirconia, diamond, glass, ruby, sapphire, zinc oxide, boron nitride, zirconia toughened alumina, silicon dioxide, silicon carbide, silicon nitride, and combinations thereof. High-temperature glasses can include one or more of boro-alumino, boro-aluminosilicate, and/or borosilicate-type glasses with different ranges of thermal expansions to approximately match the conductive materials used to match metallic ferrule 11, which can include niobium, titanium, titanium alloys (such as niobium-titanium, titanium-6Al-4V or titanium-vanadium), platinum, molybdenum, zirconium, tantalum, vanadium, tungsten, iridium, palladium, nickel super alloys, nickel-chromium-cobalt-molybderum alloys, and mixtures and combinations thereof. In exemplary embodiments of the present teachings, the liquid-phase or solid state sintered and co-fired ceramic insulators 20 typically have a coefficient of thermal expansion 10-20% lower than that of the conductive material used in ferrule 11.

Method Of Making A Co-Fired Ceramic Insulator

In exemplary embodiments, ceramic insulators 20 can be manufactured by applying tape casted green sheets of alumina mounted on frames. Through hole vias are punched, vias can be filled with a platinum metal paste, and surface metallization is screen printed. Other metallization materials are iridium, molybdenum, niobium, palladium, tantalum, titanium, tungsten, or combinations thereof. Individual sheets can be laminated, and subsequently fired. Next, dicing can be used to separate individual parts. Semi-circular ends can be manufactured by grinding. Infeed ultra-precision grinding can be used to obtain a partially effective insulator surface on a CNC grinder Absolute Grinding Company Inc., Cleveland, OH, USA, using a Studer S35 grinder according to manufacturer's specifications and instructions. Optionally, surface cracks greater than 30 $\mu$m can be removed by ultrafine polishing achieving a surface roughness of less than 1.5 $\mu$m. Additionally, the bulk material should be free of flaws greater than 30 $\mu$m, as they can be exposed by machining operations.

Insulator 20 may be polished using any commercial polishing machine, such as a polishing machine commercially available (for example, Struers RotoPol 35, Struers Inc., Cleveland, OH, USA) to the desired/specified surface quality (i.e., having no surface cracks at least at the brazing region of insulator 20 having a crack size greater than the critical flaw size as calculated according to Equation 3, described above). Polishing of a ceramic feedthrough insulator 20 can be conducted at a cloth disc rotation of 150 rpm and a sample rotation of 40 rpm, respectively. Diamond suspensions of 3 $\mu$m and 0.05 $\mu$m (Kemet International Limited, Maidstone, Kent, UK) together with an ethanol-containing high-quality lubricant (DP-Lubrication Blue, Struers Inc., Cleveland, OH, USA) can be consecutively applied. The applied contacting force between insulator 20 and the cloth can vary from about 20 N to about 80 N. The polishing can be conducted for a period of time ranging from about 1 minute to about 60 minutes.

High-temperature glass insulators 20 can be polished using a commercially suggested polishing procedure, including rough polishing, intermediate polishing and final polishing for 30 seconds at each step. Three different abrasive papers on a schedule of decreasing abrasive sizes can be applied. 3 $\mu$m SiC paper with an air-cushion metal pad may be applied for rough polishing, 0.1 $\mu$m diamond paper with a metal-flat pad may be applied for intermediate polishing, and 0.05 $\mu$m alumina paper with a rubber-flat pad may be applied for final polishing. In each step, 91% volume isopropyl alcohol may be applied as a coolant. Sharp edges can be rounded in a tumbling process.

The above polishing process achieves a surface quality having no cracks larger than the desired critical flaw size of 70 $\mu$m or less on surface 18 and 22 of the shaped insulator 20 to be brazed. Results of the polishing step can be verified using any one or more surface analysis tools, including confocal microscopy, electrical capacitance, electron microscopy and interferometer analysis. Further polishing can be undertaken to remove any surface cracks, for example, at the brazing joint region greater than the critical flaw size of 70 $\mu$m, at least for ceramic insulators comprising aluminum oxide. Cracks smaller than 70 $\mu$m may need to be removed if the crack size is greater than the critical flaw size for that particular insulator material when considering the insulator's acting stress.

Methods Of Making A Ceramic-Metal Feedthrough

Methods for making implantable electronic medical device feedthroughs are known in the art. For example, U.S. Patent Nos. 6,903,268 and 6,951,664 to Marshall et al. and 6,855,456 to Taylor et al. illustrate various methods of forming and manufacturing biocompatible high-temperature material feedthroughs. The exemplary feedthroughs 10 can incorporate liquid-phase or solid state sintered or co-fired ceramic insulators and high-temperature glass insulators. In addition, clean co-fired ceramic insulators can be coated with a metallic film on braze surface 18 and 22 that provide wetting of the braze metal. Regardless, ceramic insulator 20 of the present teachings should have a surface roughness ranging between 1.0 $\mu$m to 1.5 $\mu$m before being placed in or on metallic ferrule 11, and before a solid gold preform (i.e., braze material 30) is added. Thereafter, assembly 10 can be heated to a predetermined temperature (e.g., heated in a vacuum high-temperature furnace to slightly above melting temperature of the braze material 30, which may be commercially pure gold). Finally, the furnace is cooled to room temperature and the finished assemblies 10 are removed.

Methods Of Using Biocompatible Non-Conductive High-Temperature Insulators

The biocompatible non-conductive high-temperature insulators of the present teachings offer advantages in manufacturing feedthrough assemblies having diminished capacities for hermetic leaks, especially after the insulator, ferrule and electrical contacts have been brazed and thermally stressed. Generally, the implantable electronic medical devices of the present teachings can incorporate various feedthrough assembly designs, including feedthrough assemblies having one or multiple electrical contacts (e.g., two or more), even in array formats. Having multiple electrical contacts that are electromagnetically interference shielded using the feedthrough assemblies of the present teachings enables sophisticated electrophysiological applications, such as for sensing physiological parameters, for use as stimulation electrodes, *etc.* Pacemakers are most commonly operated in conjunction with one or more leads, for conveying cardiac stimulating pulses from the pacemaker to the patient's heart, and for conveying electrical cardiac signals from the heart to the pacemaker's sensing circuitry. At least two different types of pacemaker leads, unipolar and bipolar, are commonly known and used.

The foregoing description of the embodiments has been provided for purposes of illustration and description. It is not intended to be exhaustive. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the invention, and all such modifications are intended to be included within the scope of the invention as defined by the claims.

**Claims**

1. A feedthrough assembly (10) comprising:

   a metallic ferrule (11);
   a biocompatible, non-conductive, high-temperature, co-fired ceramic (HTCC) insulator (20) engaged with said metallic ferrule (11) at an interface between said ferrule and said insulator, said insulator (20) including a first surface (18) at said interface and a second surface (22) internal to said insulator (20); and
   at least one conductive member (50) disposed at said second surface (22),
   wherein at least said first surface (12) of said insulator is devoid of surface cracks greater than 30 $\mu$m.

2. The feedthrough assembly of claim 1, further comprising a braze material (30) at said interface between said ferrule (11) and said insulator (20) that hermetically seals said interface, said braze material (30) including a material having a melting point less than melting points of said ferrule (11), said conductive member, and said insulator.

3. The feedthrough assembly of claim 1, wherein said insulator (20) comprises at least one selected from the group consisting of a ceramic material, a high-temperature glass, and combinations thereof.

4. The feedthrough assembly of claim 1, wherein said insulator (20) comprises at least one selected from the group consisting of alumina, co-fired alumina, boron nitride, diamond, glass, ruby, sapphire, silicon carbide, silicon nitride, silicon dioxide, zircon, zirconia, zirconia toughened alumina, and combinations thereof.

5. The feedthrough assembly of claim 1, wherein said conductive member (50) comprises at least one selected from the group consisting of iridium, molybdenum, niobium, palladium, platinum, tantalum, titanium, tungsten, or combinations thereof.

6. The feedthrough assembly of claim 1, wherein said ferrule (11) comprises at least one selected from the group consisting of niobium, titanium, niobium-titanium alloy, titanium-6Al-4V alloy, titanium-vanadium alloy, platinum, iridium, molybdenum, zirconium, tantalum, vanadium, tungsten, palladium, nickel super alloy, nickel-chromium-cobalt-molybdenum alloy, and alloys, mixtures, and combinations thereof.

7. The feedthrough assembly of claim 1, wherein at least said first surface has a surface roughness less than 0.5 $\mu$m.

8. A medical device comprising:

   a hermetically sealed housing (102);
   a connector module (104) for connecting leads to electrical components internal to said housing; and
   the feedthrough assembly (10) of claim 1, the feedthrough assembly being located between said connector module and said housing, and connecting said leads to said electrical components.

9. A method for making a feedthrough assembly (10) for an implantable electronic medical device, the method comprising:

   polishing at least an outer surface (12) of a biocompatible, non-conductive, high-temperature, co-fired ceramic (HTCC) insulator (20) so that said outer surface (12) is devoid of surface cracks greater than 30 $\mu$m;
   providing a metallic ferrule (11) having an outer surface and a lumen surface;
   disposing said insulator (20) within said metallic ferrule (11) such that said outer surface (12) is disposed at said lumen surface to provide an interface between said ferrule (11) and said insulator (20); and
   brazing said lumen surface and at least a portion of said outer surface (12) with a braze material (30).

10. The method of claim 9, further comprising polishing said outer surface of said insulator so that said outer surface has a surface roughness less than 0.5 $\mu$m.

11. The method of claim 9, wherein said polishing step includes a rough polishing step, an intermediate polishing step, and a fine polishing step.

12. The method of claim 11, wherein said rough polishing step includes polishing said insulator with 3 $\mu$m SiC paper, said intermediate polishing step includes polishing said insulator with 0.1 $\mu$m diamond paper after said rough polishing step, and said fine polishing step includes polishing said insulator with 0.05 $\mu$m alumina paper after said intermediate polishing step.

13. The method of claim 9, wherein said polishing step includes polishing said insulator with a polishing machine.

14. The method of claim 13, wherein said polishing machine polishes said insulator using diamond suspensions of 3 $\mu$m and 0.05 $\mu$m, consecutively.


**Patentansprüche**

1. Durchführungsbaugruppe (10), mit:

   einer Metallhülse (11);
   einem biokompatiblen, nichtleitfähigem, Hochtemperatur-Mehrlagenkeramik-(HTCC)-Isolator (20), der in Eingriff mit dem Metallring (11) an einer Schnittstelle zwischen dem Ring und dem Isolator steht, wobei der Isolator (20) eine erste Oberfläche (18) an der Schnittstelle und eine zweite Oberfläche (22) intern zu dem Isolator (20) aufweist; und

zumindest ein leitfähiges Element (50), das an der zweiten Oberfläche (22) angeordnet ist, wobei zumindest die erste Oberfläche (18) des Isolators frei von Oberflächenrissen größer als 30 μm ist.

2. Durchführungsbaugruppe (10) nach Anspruch 1, ferner mit einem Lötmaterial (30) an der Schnittstelle zwischen dem Ring (11) und dem Isolator (20), der die Schnittstelle hermetisch abdichtet, wobei das Lötmaterial (30) ein Material mit einem Schmelzpunkt aufweist, der kleiner als der Schmelzpunkt des Rings, des leitfähigem Elements und des Isolators ist.

3. Durchführungsbaugruppe (10) nach Anspruch 1, wobei der Isolator (20) zumindest ein Material aus der Gruppe, die aus Keramikmaterial, Hochtemperaturglas oder Kombinationen hiervon besteht, aufweist.

4. Durchführungsbaugruppe (10) nach Anspruch 1, wobei der Isolator (20) zumindest ein Material aus der Gruppe, die aus Aluminiumoxid, Hochtemperatur-Mehrlagen-Aluminiumoxid, Bornitrid, Diamant, Glass, Rubin, Saphir, Siliziumkarbid, Siliziumnitrid, Siliziumdioxid, Zirkon, Zirkoniumdioxid, Zirkoniumdioxid verstärktes Aluminiumoxid bzw. ZTA oder Kombinationen hiervon besteht, aufweist.

5. Durchführungsbaugruppe (10) nach Anspruch 1, wobei das leitfähige Element (50) zumindest ein Material aus der Gruppe, die aus Iridium, Molybdän, Niob, Palladium, Platin, Tantal, Titan, Wolfram oder Kombinationen hiervon besteht, aufweist.

6. Durchführungsbaugruppe (10) nach Anspruch 1, wobei der Ring (11) zumindest ein Material aus der Gruppe, die aus Niob, Titan, Niob-Titanlegierung, Titan-Legierung Ti-6Al-4V, Titan-Vanadium-Legierung, Platin, Iridium, Molybdän; Zirkonium, Tantal, Vanadium, Wolfram, Palladium, Nickel-Superlegierung, NickelChrom-Kobalt-Molybdänlegierung, oder Legierungen, Mischungen oder Kombinationen hiervon besteht, aufweist.

7. Durchführungsbaugruppe (10) nach Anspruch 1, wobei zumindest die erste Oberfläche eine Oberflächenrauigkeit von weniger als 0,5 μm aufweist.

8. Medizinische Vorrichtung, mit:

   einem hermetisch abgedichteten Gehäuse (102);
   einem Anschlussmodul (104) zum Anschließen von Anschlussleitungen an elektrische Komponenten, die intern zu dem Gehäuse angeordnet sind; und
   der Durchführungsbaugruppe (10) nach Anspruch 1, wobei die Durchführungsbaugruppe zwischen dem Anschlussmodul und dem Gehäuse angeordnet ist, wobei die Durchführungsbaugruppe die Leitungen mit den elektrischen Komponenten verbindet.

9. Verfahren zum Herstellen einer Durchführungsbaugruppe (10) für eine implantierbare medizinische Vorrichtung, wobei das Verfahren aufweist:

   Polieren zumindest einer Außenoberfläche (12) eines biokompatiblen, nichtleitfähigen, Hochtemperatur-Mehrlagenkeramik-(HTCC)-Isolators (20), so dass die Außenoberfläche (12) des Isolators frei von Oberflächenrissen größer als 30 μm ist;
   Bereitstellen einer Metallhülse bzw.-eines Metallrings (11) mit einer Außenoberfläche und einer Lumenoberfläche;
   Anordnen des Isolators (20) innerhalb des Metallrings (11), derart, dass die Außenoberfläche (12) an der Lumenoberfläche angeordnet ist, um eine Schnittstelle zwischen dem Ring (11) und dem Isolator (20) bereitzustellen; und
   Verlöten der Lumenoberfläche und zumindest eines Abschnitts der Außenoberfläche (12) mit einem Lötmaterial (30).

10. Verfahren nach Anspruch 9, ferner mit Polieren der Außenoberfläche des Isolators, derart, dass die Außenoberfläche eine Oberflächenrauigkeit von weniger als 0,5 μm aufweist.

11. Verfahren nach Anspruch 9, wobei der Polierschritt einen Grobpolierschritt, einen Zwischenpolierschritt und einen Feinpolierschritt aufweist.

12. Verfahren nach Anspruch 11, wobei der Grobpolierschritt ein Polieren des Isolators mit 3 μm SiC-Papier, der

Zwischenpolierschritt ein Polieren des Isolators mit 0,1 $\mu$m Diamantpapier nach dem Grobpolierschritt, und der Feinpolierschritt ein Polieren des Isolators mit 0,5 $\mu$m Aluminiumpapier nach dem Zwischenpolierschritt aufweist.

13. Verfahren nach Anspruch 9, wobei der Polierschritt ein Polieren des Isolators mit einer Poliermaschine aufweist.

14. Verfahren nach Anspruch13, wobei die Poliermaschine den Isolator unter Verwendung von 3 $\mu$m und 0,05 $\mu$m Diamantsuspensionen nacheinander poliert.

**Revendications**

1. Ensemble de traversée (10) comprenant :

   une virole métallique (11) ;
   un isolant en céramique co-cuit, à haute température, non-conducteur, biocompatible (HTCC) (20) en prise avec ladite virole métallique (11) au niveau d'une interface entre ladite virole et ledit isolant, ledit isolant (20) comprenant une première surface (18) au niveau de ladite interface et une seconde surface (22) interne audit isolant (20) ; et
   au moins un élément conducteur (50) disposé au niveau de ladite seconde surface (22),
   dans lequel au moins ladite première surface (18) dudit isolant est dépourvue de fissures de surface supérieures à 30 $\mu$m.

2. Ensemble de traversée selon la revendication 1, comprenant en outre un matériau de brasage (30) au niveau de ladite interface entre ladite virole (11) et ledit isolant (20) qui rend hermétiquement étanche ladite interface, ledit matériau de brasage (30) comprenant un matériau ayant un point de fusion inférieur à des points de fusion de ladite virole (11), dudit élément conducteur et dudit isolant.

3. Ensemble de traversée selon la revendication 1, dans lequel ledit isolant (20) comprend au moins un élément choisi dans le groupe constitué d'un matériau en céramique, d'un verre à haute température, et de combinaisons de ceux-ci.

4. Ensemble de traversée selon la revendication 1, dans lequel ledit isolant (20) comprend au moins un élément choisi dans le groupe constitué par l'alumine, l'alumine co-cuit, le nitrure de bore, le diamant, le verre, le rubis, le saphir, le carbure de silicium, le nitrure de silicium, le dioxyde de silicium, le zircon , la zircone, l'alumine renforcée de zircone, et des combinaisons de ceux-ci.

5. Ensemble de traversée selon la revendication 1, dans lequel ledit élément conducteur (50) comprend au moins un élément choisi dans le groupe constitué par l'iridium, le molybdène, le niobium, le palladium, le platine, le tantale, le titane, le tungstène, ou des combinaisons de ceux-ci.

6. Ensemble de traversée selon la revendication 1, dans lequel ladite virole (11) comprend au moins un élément choisi dans le groupe constitué par le niobium, le titane, un alliage de niobium-titane, un alliage de titane-6Al-4V, un alliage titane-vanadium, le platine, l'iridium, le molybdène, le zirconium , le tantale, le vanadium, le tungstène, le palladium, un super-alliage de nickel, un alliage nickel-chrome-cobalt-molybdène, et des alliages, des mélanges et des combinaisons de ceux-ci.

7. Ensemble de traversée selon la revendication 1, dans lequel au moins ladite première surface a une rugosité de surface inférieure à 0,5 $\mu$m.

8. Dispositif médical comprenant :

   un boîtier hermétiquement étanche (102) ;
   un module de connecteur (104) pour la connexion de fils à des composants électriques internes audit boîtier ;
   et l'ensemble de traversée (10) selon la revendication 1,
   l'ensemble de traversée étant situé entre ledit module de connecteur et ledit boîtier, et reliant lesdits fils auxdits composants électriques.

9. Procédé de fabrication d'un ensemble de traversée pour un dispositif médical électronique implantable, le procédé comprenant les étapes consistant à :

polir au moins une surface extérieure (12) d'un isolant en céramique co-cuit à haute température, non-conducteur, biocompatible (HTCC) (20) de sorte que ladite surface extérieure (2) est dépourvue de fissures de surface supérieures à 30 $\mu$m;

fournir une virole métallique (11) ayant une surface extérieure et une surface de lumière ;

disposer ledit isolant (20) à l'intérieur de ladite virole métallique (11) de telle sorte que ladite surface extérieure (12) est disposée au niveau de ladite surface de lumière pour fournir une interface entre ladite virole (11) et ledit isolant (20), et

soumettre à un brasage ladite lumière de surface et au moins une partie de ladite surface extérieure (12) avec un matériau de brasage (30).

**10.** Procédé selon la revendication 9, comprenant en outre le polissage de ladite surface extérieure dudit isolant de sorte que ladite surface externe présente une rugosité de surface inférieure à 0,5 $\mu$m.

**11.** Procédé selon la revendication 9, dans lequel ladite étape de polissage comprend une étape de polissage grossier, une étape de polissage intermédiaire et une étape de polissage fin.

**12.** Procédé de la revendication 11, dans lequel ladite étape de polissage grossier comprend de polir ledit isolant avec du papier SiC de 3 $\mu$m, ladite étape de polissage intermédiaire comprend de polir ledit isolant avec du papier diamant de 0,1 $\mu$m après ladite étape de polissage grossier, et ladite étape de polissage fin comprend de polir ledit isolant avec du papier alumine de 0,5 $\mu$m après ladite étape de polissage intermédiaire.

**13.** Procédé selon la revendication 9, dans lequel ladite étape de polissage consiste à polir ledit isolant avec une machine à polir.

**14.** Procédé selon la revendication 13, dans lequel ladite machine à polir polit ledit isolant en utilisant des suspensions en diamant de 3 $\mu$m et 0,05 $\mu$m, consécutivement.

FIG. 1

FIG. 2

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6414835 B
- US 4678868 A, Kraska
- US 5735884 A, Thompson
- US 7164572 B
- US 7064270 B
- US 6855456 B
- US 64148835 B
- US 5175067 A
- US 20060247714 A
- US 6903268 B
- US 6951664 B, Marshall

**Non-patent literature cited in the description**

- **GRIFFITH, A. A.** The Phenomena of Rupture and Flow in Solids. *Philosophical Transactions, Series A,* 1920, vol. 221, 163-198
- **HARASAKI, A. et al.** Improved Vertical Scanning Interferometry. *Appl. Opt.,* 2000, vol. 39, 2107-2115